# EUROPEAN PATENT SPECIFICATION

(11) **EP 3 512 433 B1**
(45) Date of publication and mention of the grant of the patent: **07.06.2023**
(21) Application number: 17771380.7
(22) Date of filing: 12.09.2017
(51) Int. Cl.: A61B 10/00, A61B 10/04

(54) **FLUID SAMPLING DEVICE**
VORRICHTUNG FÜR FLÜSSIGKEITSPROBENAHME
DISPOSITIF D'ÉCHANTILLONNAGE DE FLUIDE

(30) Priority: 15.09.2016 US 201662395163 P
(43) Date of publication of application: 24.07.2019
(73) Proprietor: Biora Therapeutics, Inc., San Diego, CA 92122 (US)
(72) Inventor: KERKHOF, Klaas, 5672RH Nuenen (NL); IORDANOV, Venzteslav P., 5551ZJ Valkenswaard (NL); SHIMIZU, Jeffrey A., Poway, CA 92064 (US)
(74) Representative: Mewburn Ellis LLP
(86) International application number: PCT/EP2017/072912
(87) International publication number: WO 2018/050647

(56) References cited:
- WO-A1-2016/042302
- US-A- 4 239 040
- US-A- 5 167 626
- US-A1- 2015 011 874

## Description

### BACKGROUND

Diseases of the gastrointestinal (GI) tract are common. Moreover, the composition and health of the GI tract are increasingly implicated in a wide range of disease conditions. Thus, the examination and characterization of the GI environment is of high interest. However, the GI tract is difficult to access, particularly the small intestinal section. The most common technique for examining the GI tract is through the use of endoscopic instruments, either from the mouth or nasal cavity or from the anus. But, such procedures are invasive, uncomfortable and require a trained physician to operate. Further, more distal regions of the small intestines are not accessible without very complex instrumentation and procedures.

Capsule endoscopy (CE) has emerged as an alternative to conventional GI tract examination methods. With CE, a swallowable electronic capsule passes naturally in the GI tract while images are taken and transmitted wirelessly to an external receiver.

However, to properly diagnose and study the health of the gut it is often useful to aspirate fluid samples for analysis - imaging is not sufficient. Fluid samples may be analyzed for the presence of cells, enzymes, biomarkers, metabolome, and/or microbiota, for example. One particular area of promising research is in the examination of gut microbiota and its relation to health and disease. Specifically, there are numerous studies showing a connection of bacterial dysbiosis to disease state. Further there are many approaches towards treating disease by control of gut bacterial compositions. To date a large majority of work has been via the examination of fecal microbiota. However, it is well known that the bacterial composition in the small intestines differs from that of the feces. Further, the connection of microbiome to health and disease for many conditions is likely to be more overt in the small intestines.

Some conventional sampling devices for sampling liquids in the GI tract are known. However such devices are one or more of impractical, overly complex, and/or prohibitively expensive. For these reasons a convenient, non-invasive, reliable, and low-cost method for sampling fluids through the GI tract is needed. US 4,239,040A discloses a capsule for discharging drugs into a love body or collecting samples from the body and comprises an external cylinder having slidably mounted internal cylinder. The pre-amble of claim 1 is based on this document. US 2015/011874A1 discloses a device and method for an ingestible medical device with a rotatable element. US 5167626A discloses a medical capsule device for releasing a substance at a defined location in the gastrointestinal tract. WO 2016/042302A1 discloses a sampling device suitable for collecting a sample in an aquaculture environment, enclosed system or in the gastrointestinal tract of a human or an animal.

### SUMMARY

This application describes fluid sampling devices configured to sample fluids in an environment. For example, a fluid sampling device according to this disclosure may be configured as a swallowable capsule that samples fluids in the GI tract. In some instances, capsules according to this disclosure may be selectively controlled to sample fluids in predetermined regions of the GI tract. An aspect of the invention is the subject matter recited in claim 1. Optional features of the invention are set out in the dependent claims.

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter.

### BRIEF DESCRIPTION OF THE DRAWINGS

The present invention will become more fully understood from the detailed description and the accompanying drawings, wherein:
FIG. 1A is a cross-sectional view of a fluid sampling device in a first orientation according to an example implementation of this disclosure;
FIG. 1B is a cross-sectional view of the example fluid sampling device shown in FIG. 1A, in a different orientation;
FIG. 2 is a cross-sectional view of a fluid sampling device according to another example implementation of this disclosure;
FIG. 3 is a cross-sectional view of a fluid sampling device according to another example implementation of this disclosure;
FIG. 4 is an elevational view of a fluid sampling device according to another example implementation of this disclosure;
FIG. 5A is a cross-sectional view of a fluid sampling device in a first orientation according to another example implementation of this disclosure;
FIG. 5B is a cross-sectional view of the example fluid sampling device shown in FIG. 5A, in a different orientation;
FIG. 6 is a schematic, cross-sectional view of a fluid sampling device according to an additional example that is not an embodiment of this disclosure;
FIG. 7 is a schematic representation of an example threshold sensor that may be used in fluid sampling devices such as those illustrated in FIGS. 1A, 1B, 2-4, 5A, 5B, and 6;
FIG. 8 is a schematic representation of an example threshold sensor that may be used in fluid sampling devices such as those illustrated in FIGS. 1A, 1B, 2-4, 5A, 5B, and 6; and
FIG. 9 is a schematic representation of an example threshold sensor that may be used in fluid sampling devices such as those illustrated in FIGS. 1A, 1B, 2-4, 5A, 5B, and 6.

### DETAILED DESCRIPTION

This disclosure describes improved fluid sampling devices and systems, including swallowable capsules with incorporated fluid sampling capabilities. In some implementations of this disclosure, a fluid sampling device includes an outer housing formed as a capsule, i.e., configured for swallowing by a patient, such as a mammalian patient. In such implementations, the fluid sampling device may be configured to sample fluids in the GI tract. In other embodiments, fluid sampling devices according to this disclosure may configured for insertion into a bodily cavity, or may be implanted or otherwise placed in the body.

In some implementations, a swallowable capsule according to this disclosure may include an outer housing defining a capsule volume and an inner housing defining a sampling reservoir. The inner housing may be movable within the capsule volume, relative to the outer housing. For example, the inner housing may be movable along an axis of the capsule or rotatable about the axis. In some examples, the inner housing may be selectively moveable between a sampling position, e.g., in which fluid passes through the outer housing and the inner housing to enter the sampling reservoir, and a sealed position, e.g., in which fluid does not enter and/or exit the sampling reservoir.

In some embodiments, the inner housing may be biased toward the sealed position, e.g., by a biasing member applying a biasing force on the inner housing, inside the housing. An actuator may be positioned to provide a force that counters and overcomes the biasing force, to move the inner housing to the sampling position. For instance, the biasing member may be a spring or a deformable material. Moreover, the actuator may be a gas generator, an electro-mechanical actuator, or a mechanical actuator, by way of non-limiting example.

According to example embodiments of this disclosure, an interior of the inner housing, e.g., a sampling reservoir, is placed in fluid communication with an exterior environment of the capsule when the inner housing is in the sampling position. In some embodiments, an opening may be disposed through an outer housing comprising the exterior of the capsule and a passageway through the inner housing is connected to the opening in the sampling position. In some examples, a hollow needle or similar feature may be in fluid communication with the opening in the exterior of the housing. When the inner housing is pressed against the biasing force the hollow needle pierces a section of the inner housing such that fluid exterior to the capsule passes through the opening and the hollow needle, into the inner housing.

Fluid sampling devices according to this disclosure also may be configured to detect a location of the device in the GI tract, for example, to take a fluid sample from a certain portion of the GI tract, e.g., the duodenum, the ileum, the colon, etc. Thus, in some instances, sampling devices according to this disclosure may include a sensor that senses a position of the device. For example, the sensor may sense a pH level of the environment surrounding the capsule. The sensor may also be operably connected to the actuator, such that upon sensing a predetermined pH level, the sensor emits a signal or otherwise triggers the actuator to move the interior housing from the sealed position to the sampling position.

This disclosure relates generally to fluid sampling devices and systems, and although systems according to this disclosure will generally be described as being useful in intra-corporal, and more specifically, swallowable, devices, the concepts and systems described herein are not so limited. For example, concepts of this disclosure may be useful in devices that are inserted or otherwise placed in any cavity from which it is desirable to take a fluid sample. Stated simply, although certain embodiments and benefits will be described, other implementations, modifications, and/or benefits will be appreciated those having ordinary skill in the art, with the benefit of this disclosure.

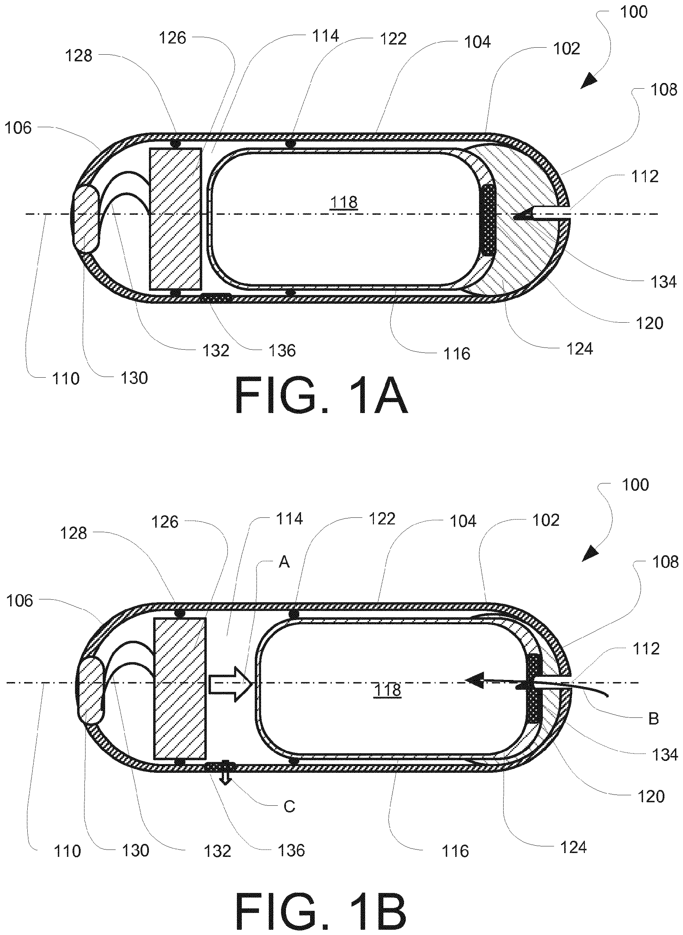

FIGS. 1A and 1B are cross-sectional representations of a fluid sampling device 100 according to embodiments of this disclosure. As will be described in more detail below, FIG. 1A depicts the fluid sampling device 100 in a sealed position and FIG. 1B depicts the device 100 in a sampling position.

The fluid sampling device 100 generally includes a housing 102 defining a size and shape of the device 100. The illustrated housing 102 is shaped like a capsule and includes a generally cylindrical sidewall 104 extending between a first end 106 and an opposite, second end 108, along an axis 110. The first end 106 and the second end 108 are generally dome-shaped to promote easier swallowing or insertion of the device 100. Of course, in other embodiments, the housing 102 may take other shapes and sizes, depending upon the application and/or the desired effect.

In some embodiments of this disclosure, the housing 102 is a rigid housing that will not substantially deform when inside a body. For example, the housing 102 may be made of one or more of many known materials, including but not limited to polymers, stainless steel, and the like. Preferably, the housing is made of a biocompatible material that is approved and/or otherwise suitable for placement in a mammalian body. For example, the housing may be formed from polyethylene, ABS, and/or PEEK.

An opening 112 is formed as a hole through the housing 102. In the illustrated example, the opening 112 is a hole formed through the second end 108 of the housing 102, generally along the axis 110. In other embodiments, the opening 112 may be offset from the axis 110 and/or may be located other than at the second end 108 of the housing 102. As will be described in more detail below, the opening 112 provides a fluid communication between the external environment of the device 100 and an inner volume 114 of the device 100, as defined by the housing 102.

As also illustrated in FIGS. 1A and 1B, an inner rigid housing 116 is disposed in the inner volume 114 of the housing 102. The inner housing 116 is generally cylindrical, also disposed along the axis 110, and defines a sealed volume comprising a sampling reservoir 116. At least a portion of the inner rigid housing comprises a resealable portion 120. As will be described in more detail below, the sampling reservoir 116 may be accessed through the resealable portion 120.

An outer circumference of the rigid housing 116, i.e., a circumference about the axis 110, is smaller than a circumference of an inner surface of the sidewall 104 of the outer housing 102. In this manner, the rigid housing 116 is movable within the housing 102, including along the axis 110. As also illustrated in FIGS. 1A and 1B, a seal 122, such as a wiper seal or an o-ring, is disposed to contact the inner surface of the sidewall 104 of the external housing 102 and an outer surface of the internal housing 116. The wiper seal 122 preferably restricts movement of the inner housing 116 in any direction other than along the axis 110. The wiper seal 122 may also partition the inner volume 114 of the device 100 into two volumes, namely, a first volume proximate the first end 106 of the device 100 and a second volume proximate the second end 108 of the device 100. In other embodiments, additional seals may be provided at other locations along an axial length of the rigid housing 116. For example, such additional seals may prevent the rigid housing 116 from pivoting about the seal 122, relative to the axis 110.

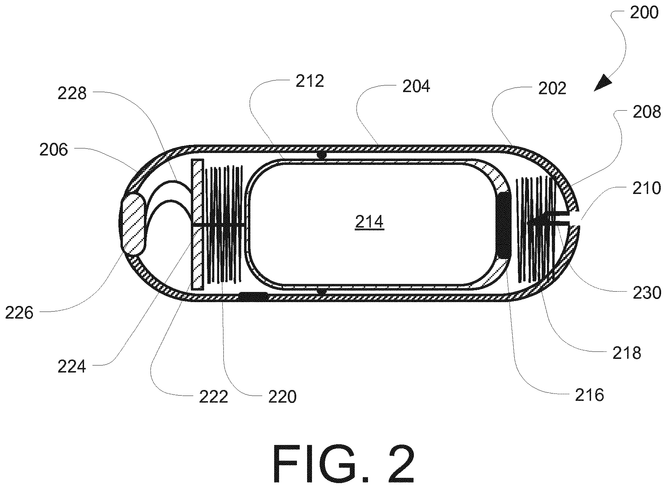

A biasing member 124 and an actuator 126 also are provided in the inner volume 114. More specifically, the biasing member is disposed proximate the second end 108 of the housing 102, and the actuator 126 is disposed proximate the first end 106 of the housing 102. For example, the biasing member 124 may comprise a compressible material that compresses under a compressive force, but expands to its original position when the compressive force is removed. In the present example, the biasing member 124 is chosen to bias the rigid housing 116 in a direction away from the second end 108 of the housing 102. That is, the biasing member maintains a predetermined spacing between the rigid housing 116 and the second end 108. For example, the compressible material may include silicon, rubber, a gel, foam, or the like. In an alternative example that is not an embodiment, such as the example illustrated in FIG. 2, the biasing member 124 may alternatively comprise a spring. Other biasing members may also be evident to those having skill in the art, with the benefit of this disclosure.

The actuator 126 is configured to apply a force to the rigid housing 116 sufficient to overcome a biasing force provided by the biasing member 124, thereby moving the rigid housing 116 toward the second end 108 of the housing 102. In the illustrated example of FIGS. 1A and 1B, the actuator 126 comprises a gas generating cell, such as a hydrogen generating cell. Also in this example, a seal 128 is provided to seal the gas generating cell 126 relative to an inner surface of the sidewall 104 of the housing 102. As will be described in more detail below, the seal 128 may be provided such that gas generated by the gas generating cell 126 is confined to an area of the internal volume 114 between the seal 128 and the seal 122 described above. In other examples that are not embodiments, including some described below, the actuator 126 may be other than a gas generating cell. For example, the actuator 126 may be a mechanical spring, such as a preloaded helical spring. In some of these examples that are not embodiments, the seal 128 may not be necessary.

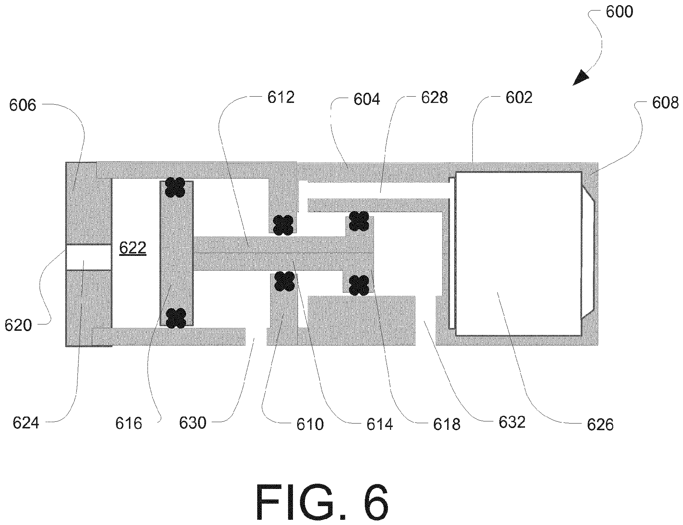

The device 100 also includes a sensor 130, proximate the first end 106 of the housing 102. The sensor 130 is operably connected to the actuator 126, e.g., via one more leads 132. The sensor may be a threshold sensor, for example, of a type that includes an enteric polymer material deposited over electrodes. Some example threshold sensors are illustrated in FIGS. 6-8, discussed below. In this example, the sensor 130, or a portion of the sensor 130, is disposed on an external surface of the housing 102. In use, a raised pH and aqueous environment may cause the enteric polymer to erode. This exposes the electrodes and this condition is used as an electrical switch. Note that the selection of polymer material, along with the possibility of layering different polymer materials, makes the switch sensitive to different pH environments and may be used to target different locations within the GI tract, for example. Although the illustrated example shows the sensor 130 disposed at the first end 106 of the housing, the sensor 130 may be disposed elsewhere on the housing 102.

The fluid sampling device 100 also includes a hollow needle 134 connected to the opening 112. The hollow needle 134 protrudes generally inwardly from the second end 108 of the housing 102 into the volume 114. As illustrated in FIG. 1A, when the device 100 is in the sealed position, the hollow needle does not protrude as far from an inner surface of the end 108 as the biasing member 124. As a result, the hollow needle does not contact the inner, rigid housing 116. However, when the biasing member is compressed toward the second end 108 of the housing 102, e.g., by the actuator 126 applying a pressure to the rigid housing 116 to move to the sampling position shown in FIG. 1B, the hollow needle may contact and pierce the resealable portion 120.

As noted above, the fluid sampling device 100 is in a sealed configuration in FIG. 1A, and in a sampling position in FIG. 1B. In operation, the fluid sampling device 100 is preferably ingested or otherwise inserted into the body in the sealed position of FIG. 1A. In one example, the device 100 is swallowed and traverses the gastrointestinal tract. The sensor 130 senses a position of the device 100 in the GI tract. As noted above, in some instances, the sensor 130 is a threshold sensor that identifies a predetermined location in the GI tract, e.g., by detecting a change in pH. In some examples, as described in more detail below, the threshold sensor may include electrodes coated with a polymer material. The polymer material may be chosen to erode at a predetermined pH level, allowing contact of the electrodes and closing a circuit across the actuator. In the illustrated embodiment of FIGS. 1A-1B, the actuator 126 is a gas generator. Accordingly, the gas generator begins to emit gas into the volume 114, generally in a direction toward the rigid housing 116, in response to the sensor 130 sensing a condition in the GI tract. In other embodiments, an electrical signal may be created when the predetermined location is sensed by the sensor. This electrical signal is conveyed to the actuator 126, e.g., via an electronic circuit, by the leads 132.

As pressure builds in the housing 102 because of the gas generated by the gas generating actuator 126, the rigid housing 116 is forced to move axially toward the second end 108 of the housing 102. This movement is generally along arrow A illustrated in FIG. 1B. Eventually, the pressure on the rigid housing 116 along the direction of arrow A is sufficient to overcome the biasing force applied in an opposite direction by the biasing member 124. More specifically, the biasing member 124 begins to compress, and with continued compression of the biasing member 124, the resealable portion 120 of the rigid housing 116 comes in contact with the hollow needle 134 and the hollow needle 134 pierces through the resealable portion 120. In this position, a passageway is created through the opening 112 and the hollow needle 134 that connects the outer environment of the device 100 with the sampling reservoir 118. In some embodiments, the sampling reservoir 118 may be under vacuum condition, such that once impermeable membrane 120 is pierced by the hollow needle, fluid external to the device 100 rushes in to fill the space that is the sampling reservoir 118. For example, the flow of fluids from the exterior of the device 100 into the sampling reservoir 118 is generally illustrated by Arrow B in FIG. 1B.

As illustrated in FIG. 1B, the housing 102 also includes an exhaust 136. The exhaust 136 may be formed as one or a plurality of apertures or holes formed through a sidewall of the housing 102. In some preferred embodiments, the exhaust 136 may comprise at least a portion of the sidewall of the housing 102 that is made of a porous material that allows for passage of gaseous contents in the internal volume 114 of the housing 102 to a position outside the housing 102. For example, some polymers are known through which vapor or gaseous materials may migrate, and a portion of the housing 102 may be formed from such a polymer to provide the exhaust. In some embodiments, a dissipation film may be made from silicone, for example, and placed over a hole or other opening formed through the housing. Microporous materials also are known, and could form a portion of the exhaust 136.

In the example, the exhaust 136 allows excess pressure in the internal volume 114 to be relieved, i.e., generally along arrow C. In this example, the exhaust 136 may comprise a vapor permeable polymer chosen to dissipate gas generated by the gas generator therethrough at a rate slower than the rate at which gas is produced (i.e., so sufficient pressure can build up in the volume 114 to move the rigid housing 116 against the biasing force). When the gas generator ceases generating gas, gas in the volume 114 escapes through the exhaust 136 until the force applied by the gas is overcome by the biasing force of the biasing member. As the biasing force moves the rigid housing 116 back to the sealed position, the hollow needle becomes disengaged from the resealable portion 120, and the resealable portion re-seals, thereby sealing the fluids that entered the sampling reservoir 118 via the hollow needle 134 in the sampling reservoir.

After the device 100 exits the GI tract, it can be retrieved so fluid in the sampling reservoir can be removed and tested.

FIG. 2 illustrates another fluid sampling device 200 according to another embodiment of this disclosure. Like the device 100, the device 200 may also be configured to be swallowed by a user, e.g., to obtain fluid samples in the GI tract. The device 200 includes a capsule-shaped housing 202, generally including a cylindrical sidewall 204 extending between a dome-shaped first end 206 and an opposite, dome-shaped second end 208. An opening 210 is illustrated as being formed through the second end, although it could be formed at other locations on the housing 202. Also similar to the device 100 illustrated in FIGS. 1A and 1B and discussed in detail above, the device 200 includes a rigid housing 212 disposed in the housing 202. The rigid housing 212 defines a sampling reservoir 214. The rigid housing 212 also includes a resealable portion 216, similar to the resealable portion 120.

In the embodiment illustrated in FIG. 2, a biasing member 218 also is provided to bias the rigid housing 212 away from the opening, and an actuator 220 is provided to selectively force the rigid housing 212 against the biasing member 218. While the biasing member could include a compressible material, as in previous examples, in this example the biasing member 218 is illustrated as a helical spring. Also, instead of the gas generator of FIGS. 1A and 1B, the actuator 220 includes a compressed helical spring and a power cell 222 that is fixed relative to the outer housing 202. The spring comprising the actuator 220 is retained in the compressed position by a frangible wire 224. The frangible wire 224 is operably connected to the power cell 222, and the power cell is in electrical communication with a sensor 226, e.g., by one or more leads 228. As in previously-discussed examples, the sensor 226 is positioned proximate the first end 206 of the housing 202, and disposed to monitor a location of the device 200. For example, the sensor may include a pH sensor that monitors the pH of the surroundings.

In operation, the device 200 may be swallowed and therefore traverses the GI tract. When the sensor 226 determines that the device 200 has reached a predetermined position, e.g., by sensing a predetermined pH level, the sensor 226 generates an electrical signal that triggers the power cell 222 to apply concentrated electrical energy to the frangible wire 224. The energy is sufficient to weaken and break the frangible wire 224, thereby releasing the compressed spring. As the spring 220 extends, contacts the rigid housing and imparts an axial motion on the rigid housing 212 with sufficient force to overcome the biasing force of the biasing member 218. As in the example described above with reference to FIGS. 1A-1B, as the rigid housing 212 continues to move toward the second end 208 of the housing 202, a hollow needle 230 pierces the resealable portion 216 of the rigid housing 212, creating a passageway from an exterior of the device 200 to the sampling reservoir 214. In some examples, the initial force created upon breaking the frangible wire 224 is sufficient to overcome the biasing force of the biasing member 218 such that the rigid housing 212 contacts the needle 230 and the sampling reservoir 214 is filled. After the initial force, however, the biasing force of the biasing member 218 may be sufficient to return the device 200 back to a sealed position. More specifically, in an equilibrium state in which the frangible wire 224 is broken, a biasing force applied on the rigid housing 212 by the biasing member 218 is sufficient to counteract the spring 222 such that the hollow needle 230 is spaced from the rigid housing 212. Only the initial force generated by releasing the compressed spring 220 is sufficient to overcome the biasing force. In this manner, a sample is collected in the sampling reservoir 214, but the device 200 returns to a sealed position, which will prevent additional fluids from entering the fluid sampling reservoir 214 via the opening 210 and the hollow needle 230.

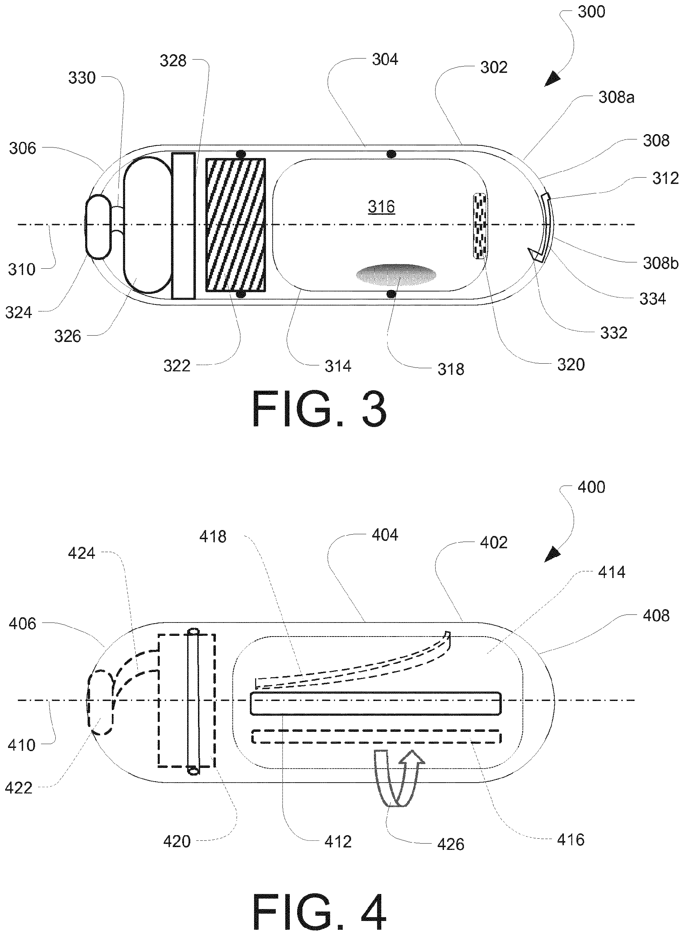

FIG. 3 illustrates another example fluid sampling device 300 according to an example that is not an embodiment of this disclosure. As illustrated in FIG. 3, the fluid sampling device 300 has many similarities to the sampling devices 100, 200 discussed above. For instance, the device 300 includes an outer housing 302 shaped like a capsule and including a generally cylindrical sidewall 304 extending between a first end 306 and the second and 308, generally along an axis 310. Moreover, an opening 312 is formed proximate the second end 308 of the housing 302. A rigid housing 314 is disposed in the volume defined by the housing 302, and the rigid housing 314 defines a fluid sampling reservoir 316. Different from previous embodiments, FIG. 3 also illustrates a quenching agent 318 disposed in the fluid sampling reservoir 316. The quenching agent 318 is placed in the fluid sampling reservoir 316 prior to use. In some examples that are not embodiments, the quenching agent is provided to prevent degradation of the sample, once collected. The quenching agent may be selected to inhibit bacterial interaction and growth with the sample. In one example, the quenching agent may include cell lysis for example to preserve the DNA content of the sample taken. Although the quenching agent is not illustrated in some other examples of this disclosure, a quenching agent could be used in any of the fluid sampling reservoirs described herein.

The rigid housing 314 also includes a resealable portion 320, and the device 300 includes an actuator 322, which may be a gas generating cell, a mechanical actuator, and/or an electromechanical actuator, for example. The device 300 also includes a sensor 324 connected to a power source 326 and electronics 328, e.g., by leads 330. In this example, the sensor 324 may be different from the threshold sensors discussed above. For example, the sensor 324 may be an electronic-based pH sensor, such as an ISFET. Unlike the threshold sensors described above, which may comprise a simple short across a gas generating cell, the electronics 328 associated with the sensor 324 may include a timer, a microcontroller, and/or wireless communication components. The power source 326 may be batteries, or the like.

Although the structure of the actuator 322, sensor 324, and additional, related components may be different from the example discussed above, the effect is generally the same. For instance, the sensor is disposed to sense a position, location, or predetermined environmental factor, and upon that sensing, the actuator 322 is driven to move the rigid housing 314 generally along an axis 310 toward the second end 308 of the housing 302. As with previous embodiments, a hollow needle 332 is disposed in fluid communication with the opening 312, and as the rigid housing 314 is driven toward the second end 308, the hollow needle 332 will pierce through the resealable portion 320 of the rigid housing 314, thereby allowing fluid external to the device 300 to enter the fluid sampling reservoir 318. Unlike other embodiments, however, the hollow needle 332 is offset relative to the opening 312 by a channel 334 disposed in the end 308 of the housing 302. In the illustrated example, the channel 334 is formed as a path in the thickness of the material comprising the second end 308. More specifically, the channel is disposed between an inner surface 308a and outer surface 308b of the second end 308 of the housing 302. The channel 334 may comprise a serpentine or other tortuous path that may provide an increased resistance to flow, thereby extending the duration required of the sampling process. Moreover, the flow path may provide a diffusion barrier and improved isolation of the sampling chamber relative to the outside environment. As illustrated, the channel 334 may obviate the need for a biasing member such as those described above. In other examples that are not embodiments, however, a biasing member such as the biasing member 124 or the biasing member 218 described above may be used. When a biasing member is used, the hollow needle 332 may be required to extend further away from the inner surface 308a of the housing 302 than illustrated in FIG. 3.

FIG. 4 illustrates yet another example of a fluid sampling device 400 according to another embodiment of this disclosure. Similar to previous embodiments, the fluid sampling device 400 includes an outer housing 402 shaped generally as a capsule. The housing 402 includes a generally cylindrical sidewall 404 for extending between a first end 406 and an opposite, second end 408, generally along an axis 410. In this example, an opening 412 is formed through the housing 402. However, instead of being a generally axial opening arranged at an end of the housing 402, the opening 412 is illustrated as a slot or similar elongate opening formed through the sidewall 404 of the housing 402. As in previous embodiments, a rigid housing 414 is disposed in the volume defined by the housing 402 and is configured to move relative to the outer housing 402. An opening 416 is disposed in a sidewall of the rigid housing 414. The opening 416 is generally shaped and sized to correspond to the opening 412 in the outer housing 402. Also in this embodiment, a propeller or fin 418 is disposed on an exterior of the inner, rigid housing 414. The fin 418 protrudes radially outwardly from an outer surface of the rigid housing 414 at an angle relative to the axis 410.

The device 400 also includes an actuator, illustrated as a gas generator 420. The gas generator 420 is electrically connected to a sensor 422 for example, by leads 424. The gas generator 420 and the sensor 422 may be similar to those discussed above with reference to other embodiments of this disclosure. They generally function in the manner described above. More specifically, the sensor 422 is configured to sense a predetermined condition of an environment of the device 400, and upon sensing that condition, the gas generating cell 420 generates a gas. The gas is forced from the gas generating cell 420 generally in a direction along the axis 410, from the gas generating cell 420 toward the second end 408 of the housing 402. The gas preferably contacts the fin 418, which is canted relative to the axial direction. Accordingly, the force of the generated gas on the fin 418 imparts a rotational motion on the housing 414, causing the housing 414 to move generally in the direction of arrow 426, relative to the outer housing 402. As the rigid housing 414 rotates relative to the outer housing 402, the opening 416 formed in the sidewall of the rigid housing 414 comes in to registration with the opening 412 formed in the sidewall 404 of the outer housing 402. Accordingly, fluid in the environment of the device 400 is allowed to enter a sampling reservoir defined by the rigid housing 414.

In some embodiments, the rigid housing 414 may continue to rotate about the axis 410 until the opening 416 is no longer in registration with opening 412 in the housing 402, thereby resealing the inner housing 416. Although not illustrated, one or more seals may be used to ensure that the fluid sample obtained by the device 400 is retained in the rigid housing 414 after collection. For instance, a wiper seal or the like that circumscribes the opening 416 may protrude radially outwardly from the exterior surface of the rigid housing 414. In this example, the wiper seal may contact an inner surface of the sidewall 404 of the housing 402 such that the sampling reservoir defined by the rigid housing 414 is sealed.

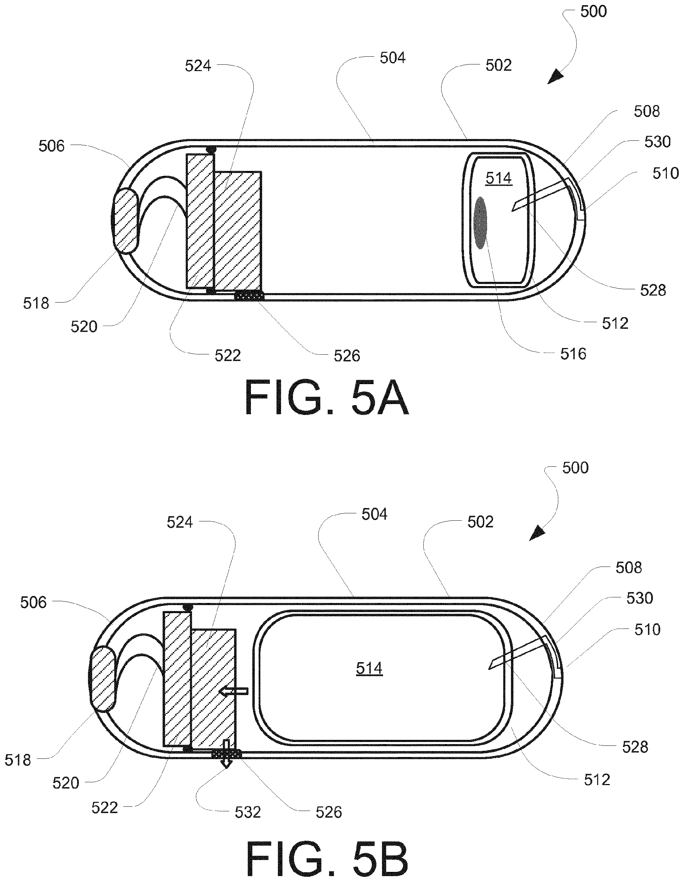

FIGS. 5A and 5B illustrate yet another embodiment of a fluid sampling device 500 according to this disclosure. The device 500 includes a housing 502 generally comprising a cylindrical sidewall 504 extending between a first end 506 and a second, opposite end 508. An opening 510 is formed in the second end 508 of the housing 502. Unlike previous embodiments, however, in the embodiment illustrated in FIGS. 5A and 5B, the rigid inner housing is replaced with an expandable bladder 512. The expandable bladder 512 may be a collapsed bag, for example. The bladder 512 defines a fluid sampling reservoir 514, and a quenching agent 516 may be disposed in the fluid sampling reservoir 514.

The device 500 also includes a sensor 518 electrically connected, e.g., via leads 520 to a controller 522. A pump 524, such as a piezoelectric pump is operably connected to the controller 522. The pump 524 is in fluid communication with the outside of the device 500 via an exhaust 526 disposed through the sidewall 504 at a location spaced from the second end 508 of the housing 502.

The fluid sampling reservoir 514 is in fluid communication with an external environment of the device 500 through a hollow needle 528 and a fluid channel 530 in fluid communication with the opening 510.

In operation, as in previous embodiments, the sensor 518 determines when the device 500 has reached a predetermined, sampling location. Sensing the predetermined location causes the pump 524 to begin to pump contents (e.g., gas or liquid) from inside the housing 502 to a position outside the device 500, i.e., via the exhaust 526. As the air is exhausted, generally in the direction of arrow 532, negative pressure is created inside the housing 502, causing the bladder 512 to expand. As the bladder 512 expands, fluid outside the device 500 is drawn into the fluid sampling reservoir 514 via the passageway formed by the opening 510, the channel 530, and the hollow needle 528. FIG. 5B illustrates a state in which the fluid reservoir 514 is filled with an analyte solution comprising the fluid sample from the capsule exterior and mixed with the quenching agent 516. Although not illustrated, a one-way valve may be disposed in communication with the opening 510, the channel 530, or the hollow needle 528, for example, to prevent the fluid sample retained in the expanded bladder 512 from exiting back through the opening 510, when the pump is turned off.

FIG. 6 illustrates another example that is not an embodiment of a fluid sampling device 600 according to additional examples that are not embodiments of this disclosure. As in previous examples, the device 600 includes a housing 602 including a sidewall 604, which may be a cylindrical sidewall, extending between a first end 606 and a second end 608. In this example that is not an embodiment, a partition 610 is provided that generally divides the inner volume defined by the housing 602 into two chambers, one proximate the first end 606 and the other proximate the second end 608. A movable member 612 arranged for axial movement relative to the housing 602 generally includes a shaft 614 extending axially through the partition 610. A first piston 616 is positioned between the first end 606 and the partition 610 and is sealed relative to an inner surface of the sidewall 604. A second piston 618 is positioned between the second end 608 and the partition 610, and is sealed relative to an inner surface.

An opening 620 is formed through the housing 602 at the first end 606. The opening 620 fluidly connects an external environment of the device 600 with a sampling reservoir 622 via a passageway 624. In this example that is not an embodiment, the passageway is illustrated as a generally cylindrical channel extending along an axis of the device 600. The sampling reservoir 622 is defined by an inner surface of the sidewall 604, an inner surface of the first end 606, and the first piston 616.

A gas generating cell 626 is provided as an actuator proximate the second end 608 of the housing 602. A gaseous output of the gas generating cell 626 is routed via a gas passageway 628 from the gas generating cell 626 to a side of the second piston 618 opposite the second end 608. In this manner, the gas generated by the gas generating cell 626 forces the moveable member 612 toward the second end. 608. This movement of the moveable member 612 causes the sampling reservoir 622 to expand, creating a negative pressure. The negative pressure causes fluid external to the device 600 to enter the sampling reservoir 622 via the passageway 624. In some implementations, a one-way valve may be provided in communication with the passageway 624, to prevent fluid from exiting the sampling reservoir 622. As illustrated, vents 630, 632 also are provided, to allow movement of the moveable member 612.

The two-piston configuration depicted in FIG. 6 provides one specific arrangement, which outlines the principle of operation. However, there are many other configurations possible. By way of non-limiting example, the actuator may be placed in the center of the device and one or more arm(s) connecting the two pistons 616, 618 may be positioned close to the inner surface of the sidewall 604.

Although not illustrated, the device 600 may also include a sensor such as the sensors described above, to trigger the gas generating cell 626 to generate gas. The device may also include a controller and/or other electronics necessary to operation.

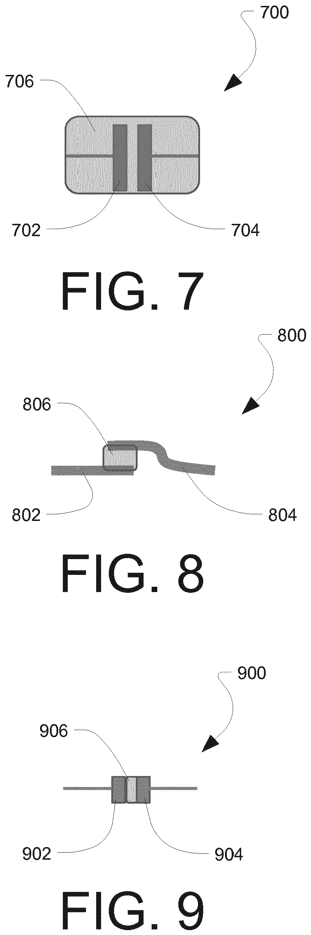

FIGS. 7-9 illustrate examples that are not embodiments of thresholds sensors that may be used in examples that are not embodiments of this disclosure. More specifically, FIG. 7 illustrates a threshold sensor 700 that includes a first metal electrode 702 and a second metal electrode 704 spaced from the first metal electrode 702. For example, the first metal electrode 702 and the second metal electrode 704 may be electrically connected to leads, such as leads 132, 228, 330, 424, 520, discussed above. A pH-sensitive material 706 is disposed over the electrodes 702, 704 to prevent a galvanic connection between the metal electrodes 702, 704. The pH-sensitive material 706 is selected to melt or otherwise dissolve at a predetermined pH level, for example, a pH-level of greater than 6. When the pH-sensitive material 606 melts or dissolves, the electrodes 702, 704 are exposed, and galvanic contact is made via the surrounding fluid. This galvanic contact may directly result in actuation of a gas generating cell, or trigger an active electronics circuit. The thickness of the pH sensitive layer may be varied in some examples that are not embodiments to create a predetermined delay to the moment of sampling. This may be useful to target different regions within the GI tract, for example.

FIG. 8 illustrates another example that is not an embodiment of a threshold sensor 800. Threshold sensor 800 includes a first electrode 802 and a second electrode 804. In this example, the second electrode 804 is biased away from the first electrode 802 by a pH sensitive material 806. The pH sensitive material 806 melts or dissolves much like the pH sensitive material 806 of the sensor 800. However, in the sensor 800, melting or dissolution of the pH sensitive material 806 results in the second electrode 804 physically moving, to make a physical contact with the first electrode 802. More specifically, the pH sensitive material 806 puts the second electrode 804 forces the second electrode 804 away from the first electrode 802, such that when the pH-sensitive material 806 no longer present, the second electrode 804 returns to its unbiased position, contacting the first electrode 802.

FIG. 9 illustrates yet another example of a threshold sensor 900 according to examples that are not embodiments of this. In FIG. 9, a first electrode 902 and a second electrode 904 are provided, spaced relative to each other. A pH-sensitive material 906 is disposed in the space between the first electrode 902 and the second electrode 904. In this example, however, the pH-sensitive material 906 is a pH-sensitive gel that absorb surrounding liquid if the pH value of that liquid exceeds a predetermined value. As the liquid is absorbed, a galvanic contact is formed between the first electrode 902 and the second electrode 904, thereby signaling that the surrounding environment of the sensor 900 has a predetermined pH value.

Sensors such as those illustrated in FIGS. 7-9 are generally used to determine that an environment of the sensor has a predetermined pH because areas of the GI tract have discernable pH variations. For instance, a pH of the stomach is markedly different from a pH of the small intestine. Accordingly, by determining the pH, a capsule can readily be configured to sample fluids in the stomach or in the small intestine. Of course, other sensors may be used to determine a position of fluid sampling devices according to this disclosure. For instance, a sensor may sense a position, e.g., using global positioning technology. In still other examples, a sensor may not be necessary at all. For instance, a clock or timer may be disposed in the fluid sampling device which may measure a time, e.g., since the capsule was swallowed, which is used to instead trigger fluid sampling.

While several of the embodiments described above discuss a swallowable fluid sampling device, in other embodiments the device may not be swallowed. For example, the fluid sampling device may be inserted into a bodily cavity, e.g., via the anus or vagina. In these embodiments, a sensor may also not be necessary. Also in these embodiments, the device may include a retrieval feature, i.e., for removing the device after retrieving the sample. For instance, a rigid or flexible structure, like a handle or string, may be provided on an exterior of the device to promote retrieval.

Various combinations of the foregoing illustrated embodiments will be appreciated by those having ordinary skill in the art. More specifically, this disclosure is not limited to the combinations of features illustrated in the Figures. By way of non-limiting example, although FIGS. 1A and 1B illustrate use of a gas generating cell with a compressible material biasing member, while FIG. 2 illustrates a spring actuator and a spring biasing member, either of those embodiments could instead include a spring actuator and a compressible member biasing member or a gas generating cell actuator and a spring biasing member. Similarly, different types of sensors, e.g., the ISFET and threshold sensors, could be used in different embodiments than those illustrated. Other combinations, although not explicitly stated herein, will be understood by those having ordinary skill in the art, with the benefit of this disclosure.

Although embodiments have been described in language specific to structural features and/or methodological acts, it is to be understood that the invention is not necessarily limited to the specific features or acts of the embodiments described. Rather, the specific features and acts are disclosed as illustrative forms of implementing the invention. For example, while embodiments are described having certain shapes, sizes, and configurations, these shapes, sizes, and configurations are merely illustrative. Also, while some example processes and uses are described, sampling devices according to this disclosure may be made and/or used differently. The scope of the invention is defined by the appended claims solely.

## Claims

1. A device (100) for sampling contents in a bodily cavity of a patient, the device (100) comprising:
an outer housing (102) comprising a sidewall (104) extending between a first end (106) and a second end (108) spaced along an axis from the first end (106) and an opening (112) extending through the sidewall (104) proximate the second end (108);
a sampling reservoir (118) comprising a rigid housing (116) defining a volume and a re-sealable portion (120), the sampling reservoir (118) being disposed in the outer housing (102) and moveable within the outer housing (102), generally along the axis, between a sealed position, relatively closer to the first end (106) of the outer housing (102), and a sampling position, relatively closer to the second end (108) of the outer housing (102);
a biasing member (124) disposed in the outer housing (102) proximate the second end (108) of the housing (102) and configured to apply a biasing force to the sampling reservoir (118) to bias the sampling reservoir (118) toward the sealed position;
**characterized in that** the device (100) comprises:
a gas generating cell (126) disposed in the outer housing (102) proximate the first end (106) of the outer housing (102) and configured to generate gas at an actuating rate sufficient to move the sampling reservoir (118) from the sealed position to the sampling position, against the biasing force;
a sensor (130) disposed to sense a condition of an environment external to the outer housing (102), the sensor (130) being electrically connected to the gas generating cell (126) to selectively control the gas generating cell (126) to generate the gas; and
a hollow needle (134) fixed relative to the outer housing (102) and in fluid communication with the opening (112) extending through the sidewall (104),
wherein the re-sealable portion (120) of the sampling reservoir (118) is pierced by the hollow needle (134) when the sampling reservoir (118) is in the sampling position and the re-sealable portion (120) of the sampling reservoir (118) is spaced from the hollow needle (134) when the sampling reservoir (118) is in the sealed position.

2. The device (100) of claim 1, further comprising an exhaust (136) in the outer housing (102) proximate the gas generating cell (126).

3. The device (100) of claim 2, wherein the exhaust (136) comprises a gas permeable membrane having a gas permeability that allows gas to flow through the gas permeable membrane at a rate lower than that actuating rate at which the gas generating cell generates gas.

4. The device (100) of claim 1, wherein the sensor (130) comprises a pH sensitive material that causes a signal at the sensor (130) when a pH of the environment exceeds a threshold pH.

5. The device of claim 1, further comprising a vent in the outer housing (102) proximate the gas generating cell.

6. The device of claim 5, wherein the vent comprises a gas permeable membrane having a gas permeability lower than a rate at which the gas generating cell (126) generates gas.

7. The device of claim 1, wherein the sensor (130) comprises a threshold sensor responsive to a threshold pH of the environment.

8. The device of claim 1, wherein the hollow needle (134) comprises a channel fluidly connecting the hollow needle (134) to the opening.

9. The device (400) of claims 5 to 8, wherein:
the sampling reservoir comprises a rigid, cylindrical housing (414), a fin (418) extending radially outwardly from an outer surface of the cylindrical housing (414), and a reservoir opening (416) formed in a sidewall of the cylindrical housing (414),
and
gas generated by the gas generating cell (420) contacts the fin (418) to rotate the sampling reservoir relative to the outer housing (402) between the sealed position, in which the opening (412) extending through the sidewall (404) of the outer housing (402) is rotationally spaced from the reservoir opening (416), and the sampling position, in which the opening (412) extending through the sidewall (404) of the outer housing (402) aligns with the reservoir opening (416).

10. The device of claims 5 to 9, wherein:
the sampling reservoir (514) comprises an expandable bag (512) in fluid communication with the opening (510),
a piezo pump (524) spaced from the expandable bag (512),
the piezo pump (524) pumps air in the outer housing (502) between the expandable bag (512) and the piezo pump (524), outside the expandable bag (512), out of the outer housing (502) via the vent (526),
the expandable bag (512) expands in response to the piezo pump (524) pumping the air out of the outer housing (502), and
expansion of the expandable bag (512) draws fluids external to the outer housing (502) into the expandable bag (512) through the opening (510).

## Patentansprüche

1. Vorrichtung (100) zur Beprobung von Inhalten eines Körperhohlraums eines Patienten, wobei die Vorrichtung (100) Folgendes umfasst:
ein Außengehäuse (102), das eine Seitenwand (104), die sich zwischen einem ersten Ende (106) und einem zweiten Ende (108), das entlang einer Achse von dem ersten Ende (106) beabstandet ist, erstreckt, und eine Öffnung (112), die sich nahe dem zweiten Ende (180) durch die Seitenwand (104) hindurch erstreckt, umfasst;
einen Beprobungsbehälter (118), der ein starres Gehäuse (116), das ein Volumen definiert, und einen wiederversiegelbaren Abschnitt (120) umfasst, wobei der Beprobungsbehälter (118) in dem Außengehäuse (102) angeordnet und innerhalb des Außengehäuses (102) im Wesentlichen entlang der Achse zwischen einer versiegelten Position, relativ näher zu dem ersten Ende (106) des Außengehäuses (102), und einer Beprobungsposition, relativ näher zu dem zweiten Ende (108) des Außengehäuses (102), bewegbar ist;
ein Vorspannelement (124), das in dem Außengehäuse (102) nahe dem zweiten Ende (108) des Gehäuses (102) angeordnet und ausgelegt ist, um eine Vorspannkraft auf den Beprobungsbehälter (118) zu beaufschlagen, um den Beprobungsbehälter (118) in Richtung der versiegelten Position vorzuspannen;
**dadurch gekennzeichnet, dass** die Vorrichtung (100) Folgendes umfasst:
eine Gaserzeugungszelle (126), die in dem Außengehäuse (102) nahe dem ersten Ende (106) des Außengehäuses (102) angeordnet und ausgelegt ist, um mit einer Betätigungsrate, die ausreichend ist, um den Beprobungsbehälter (118) gegen die Vorspannkraft von der versiegelten Position in die Beprobungsposition zu bewegen, ein Gas zu erzeugen;
einen Sensor (130), der angeordnet ist, um einen Zustand einer Außenumgebung des Außengehäuses (102) abzufühlen, wobei der Sensor (130) elektrisch mit der Gaserzeugungszelle (126) verbunden ist, um die Gaserzeugungszelle (126) selektiv anzusteuern, um das Gas zu erzeugen; und
eine Hohlnadel (134), die relativ zu dem Außengehäuse (102) fixiert und in Fluidkommunikation mit der Öffnung (112) ist, die sich durch die Seitenwand (104) hindurch erstreckt,
wobei der wiederversiegelbare Abschnitt (120) des Beprobungsbehälters (118) von der Hohlnadel (134) durchstochen wird, wenn der Beprobungsbehälter (118) sich in der Beprobungsposition befindet, und der wiederversiegelbare Abschnitt (120) des Beprobungsbehälters (118) von der Hohlnadel (134) beabstandet ist, wenn der Beprobungsbehälter (118) sich in der versiegelten Position befindet.

2. Vorrichtung (100) nach Anspruch 1, die ferner eine Ableitung (136) in dem Außengehäuse (102) nahe der Gaserzeugungszelle (126) umfasst.

3. Vorrichtung (100) nach Anspruch 2, wobei die Ableitung (136) eine gasdurchlässige Membran mit einer Gasdurchlässigkeit umfasst, die ein Strömen von Gas durch die gasdurchlässige Membran mit einer Rate ermöglicht, die geringer ist als die Betätigungsrate, mit der die Gaserzeugungszelle Gas erzeugt.

4. Vorrichtung (100) nach Anspruch 1, wobei der Sensor (130) ein pH-empfindliches Material umfasst, das ein Signal an dem Sensor (130) bewirkt, wenn ein pH der Umgebung einen Schwellen-pH übersteigt.

5. Vorrichtung nach Anspruch 1, ferner umfassend eine Entlüftung in dem Außengehäuse (102) nahe der Gaserzeugungszelle.

6. Vorrichtung nach Anspruch 5, wobei die Entlüftung eine gasdurchlässige Membran umfasst, die eine Gasdurchlässigkeit aufweist, die geringer ist als die Rate, mit der die Gaserzeugungszelle (126) Gas erzeugt.

7. Vorrichtung nach Anspruch 1, wobei der Sensor (130) einen Schwellensensor umfasst, der auf einen Schwellen-pH der Umgebung reagiert.

8. Vorrichtung nach Anspruch 1, wobei die Hohlnadel (134) einen Kanal umfasst, der die Hohlnadel (134) fluidisch mit der Öffnung verbindet.

9. Vorrichtung (400) nach Anspruch 5 bis 8, wobei
der Beprobungsbehälter ein starres, zylindrisches Gehäuse (414), eine Leitschaufel (418), die sich von einer Außenfläche des zylindrischen Gehäuses (414) radial nach außen erstreckt, und eine Behälteröffnung (416), die in einer Seitenwand des zylindrischen Gehäuses (414) ausgebildet ist, umfasst und
Gas, das von der Gaserzeugungszelle (420) erzeugt wird, die Leitschaufel (418) berührt, um den Beprobungsbehälter relativ zu dem Außengehäuse (402) zwischen der versiegelten Position, in der die Öffnung (412), die sich durch die Seitenwand (404) des Außengehäuses (402) hindurch erstreckt, rotationsmäßig von der Behälteröffnung (416) beabstandet ist, und der Beprobungsposition, in der die Öffnung (412), die sich durch die Seitenwand (404) des Außengehäuses (402) hindurch erstreckt, mit der Behälteröffnung (416) fluchtend ausgerichtet ist, zu drehen.

10. Vorrichtung nach Anspruch 5 bis 9, wobei
der Beprobungsbehälter (514) Folgendes umfasst:
einen expandierbaren Beutel (512) in Fluidkommunikation mit der Öffnung (510),
eine Piezopumpe (524), die von dem expandierbaren Beutel (512) beabstandet ist,
wobei die Piezopumpe (524) Luft in dem Außengehäuse (502) zwischen dem expandierbaren Beutel (512) und der Piezopumpe (524), außerhalb des expandierbaren Beutels (512), aus dem Außengehäuse pumpt,
wobei der expandierbare Beutel (512) als Antwort auf das Pumpen von Luft aus dem Außengehäuse (502) durch die Piezopumpe (524) expandiert und
wobei die Expansion des expandierbaren Beutels (512) Fluide außerhalb des Außengehäuses (502) über die Öffnung (510) in den expandierbaren Beutel (512) zieht.

## Revendications

1. Dispositif (100) d'échantillonnage de contenu dans une cavité corporelle d'un patient, le dispositif (100) comprenant :
un boîtier extérieur (102) comprenant une paroi latérale (104) s'étendant entre une première extrémité (106) et une seconde extrémité (108) espacées le long d'un axe à partir de la première extrémité (106) et une ouverture (112) s'étendant à travers la paroi latérale (104) à proximité de la seconde extrémité (108) ;
un réservoir d'échantillonnage (118) comprenant un boîtier rigide (116) définissant un volume et une partie pouvant être rescellée (120), le réservoir d'échantillonnage (118) étant disposé dans le boîtier extérieur (102) et mobile à l'intérieur du boîtier extérieur (102), généralement le long de l'axe, entre une position scellée, relativement plus proche de la première extrémité (106) du boîtier extérieur (102), et une position d'échantillonnage, relativement plus proche de la seconde extrémité (108) du boîtier extérieur (102) ;
un élément de sollicitation (124) disposé dans le boîtier extérieur (102) à proximité de la seconde extrémité (108) du boîtier (102) et configuré pour appliquer une force de sollicitation au réservoir d'échantillonnage (118) afin de solliciter le réservoir d'échantillonnage (118) vers la position scellée ;
**caractérisé en ce que** le dispositif (100) comprend :
une cellule de génération de gaz (126) disposée dans le boîtier extérieur (102) à proximité de la première extrémité (106) du boîtier extérieur (102) et configurée pour générer un gaz à un débit d'actionnement suffisant pour déplacer le réservoir d'échantillonnage (118) de la position scellée à la position d'échantillonnage, à l'encontre de la force de sollicitation ;
un capteur (130) disposé pour détecter une condition d'un environnement externe au boîtier extérieur (102), le capteur (130) étant connecté électriquement à la cellule de génération de gaz (126) pour commander de manière sélective la cellule de génération de gaz (126) afin de générer le gaz ; et
une aiguille creuse (134) fixée par rapport au boîtier extérieur (102) et en communication fluidique avec l'ouverture (112) s'étendant à travers la paroi latérale (104),
dans lequel la partie pouvant être rescellée (120) du réservoir d'échantillonnage (118) est percée par l'aiguille creuse (134) lorsque le réservoir d'échantillonnage (118) est dans la position d'échantillonnage et la partie pouvant être rescellée (120) du réservoir d'échantillonnage (118) est espacée de l'aiguille creuse (134) lorsque le réservoir d'échantillonnage (118) est dans la position scellée.

2. Dispositif (100) selon la revendication 1, comprenant en outre un échappement (136) dans le boîtier extérieur (102) à proximité de la cellule de génération de gaz (126).

3. Dispositif (100) selon la revendication 2, dans lequel l'échappement (136) comprend une membrane perméable au gaz présentant une perméabilité au gaz qui permet au gaz de s'écouler à travers la membrane perméable au gaz à un débit inférieur au débit d'actionnement auquel la cellule de génération de gaz génère du gaz.

4. Dispositif (100) selon la revendication 1, dans lequel le capteur (130) comprend un matériau sensible au pH qui provoque un signal au niveau du capteur (130) lorsqu'un pH de l'environnement dépasse un pH seuil.

5. Dispositif selon la revendication 1, comprenant en outre un évent dans le boîtier extérieur (102) à proximité de la cellule de génération de gaz.

6. Dispositif selon la revendication 5, dans lequel l'évent comprend une membrane perméable au gaz présentant une perméabilité au gaz inférieure à un débit auquel la cellule de génération de gaz (126) génère du gaz.

7. Dispositif selon la revendication 1, dans lequel le capteur (130) comprend un capteur de seuil sensible à un pH de seuil de l'environnement.

8. Dispositif selon la revendication 1, dans lequel l'aiguille creuse (134) comprend un canal reliant de manière fluidique l'aiguille creuse (134) à l'ouverture.

9. Dispositif (400) selon la revendication 5, dans lequel :
le réservoir d'échantillonnage comprend un réservoir cylindrique rigide (414), une ailette (418) s'étendant radialement vers l'extérieur à partir d'une surface extérieure du boîtier cylindrique (414), et une ouverture de réservoir (416) formée dans une paroi latérale du boîtier cylindrique (414), et
le gaz généré par la cellule de génération de gaz (420) vient en contact avec l'ailette (418) pour faire tourner le réservoir d'échantillonnage par rapport au boîtier extérieur (402) entre la position scellée, dans laquelle l'ouverture (412) s'étendant à travers la paroi latérale (404) du boîtier extérieur (402) est espacée en rotation de l'ouverture de réservoir (416), et la position d'échantillonnage, dans laquelle l'ouverture (412) s'étendant à travers la paroi latérale (404) du boîtier extérieur (402) est alignée avec l'ouverture de réservoir (416).

10. Dispositif selon les revendications 5 à 9, dans lequel :
le réservoir d'échantillonnage (514) comprend une poche pouvant se dilater (512) en communication fluidique avec l'ouverture (510),
une pompe piézoélectrique (524) espacée de la poche pouvant se dilater (512),
la pompe piézoélectrique (524) pompe de l'air dans le boîtier extérieur (502) entre la poche pouvant se dilater (512) et la pompe piézoélectrique (524), à l'extérieur de la poche pouvant se dilater (512), hors du boîtier extérieur (502) via l'évent (526),
la poche pouvant se dilater (512) se dilate en réponse à la pompe piézoélectrique (524) pompant l'air hors du boîtier extérieur (502), et
la dilatation de la poche (512) aspire des fluides externes au boîtier extérieur (502) dans la poche pouvant se dilater (512) à travers l'ouverture (510).
